# EUROPEAN PATENT SPECIFICATION

(11) **EP 1 954 148 B1**
(45) Date of publication and mention of the grant of the patent: **05.10.2016**
(21) Application number: 06819864.7
(22) Date of filing: 30.11.2006
(51) Int. Cl.: A23L 5/20

(54) **AQUEOUS POTASSIUM LACTATE SOLUTION**
WÄSSRIGE KALIUMLACTATLÖSUNG
SOLUTION AQUEUSE DE LACTATE DE POTASSIUM

(30) Priority: 30.11.2005 US 740680 P
(43) Date of publication of application: 13.08.2008
(73) Proprietor: PURAC Biochem BV, 4206 AC Gorinchem (NL)
(72) Inventor: VAN DIJK, Lonneke, NL-3061 ZM Rotterdam (NL); RODRIQUEZ LONGARELA, Gema, NL-4207 VC Gorinchem (NL); SINNEMA, Mirjam, NL-3011 DC Rotterdam (NL); FAHLIN, Kathleen, Blair, Nebraska 68008 (US)
(74) Representative: Beetz, Tom
(86) International application number: PCT/EP2006/069134
(87) International publication number: WO 2007/063098

(56) References cited:
- EP-A- 0 563 455
- WO-A-02/090311
- WO-A-03/031385
- GB-A- 907 321
- US-A- 2 143 361
- SYSTERMANS: "Lactates - A NewTrend in Fresh Marinated Meat" ASIA PACIFIC FOOD INDUSTRY, AP TRADE PUBLICATOINS, SINGAPORE, SG, no. 6, 2000, pages 1-2, XP002213994 ISSN: 0218-2734 & "Purasal P HiPure 60 Specification" INTERNET CITATION, [Online] XP002213995 Retrieved from the Internet: URL:http://www.purac.com/products/EN-P HiPure60PNL.PDF> [retrieved on 2002-09-19]

## Description

The invention relates to a method for making an aqueous potassium lactate solution, to the solution thus obtained, to a food or drink product containing the same, and to a method for the preparation of a food or drink product.

Aqueous potassium lactate solutions are known in the art and are commercially available. Potassium lactate is a product that is mainly used in the meat industry. It is used for the same application as sodium lactate, namely for shelf life extension and safety enhancement. The use of potassium lactate is more and more preferred since it is believed that sodium lactate can cause hypertension. A disadvantage of such aqueous potassium lactate solutions is their undesirable flavors and odors, particularly causing a bitter aftertaste.

In WO 02/090311 and EP 1385815 a process for the removal of undesirable flavors and odors from potassium lactate was described by treating the potassium lactate solution with active carbon. The resulting potassium lactate is less bitter and has a more favorable flavor and odor profile and is thus accepted by a large part of the population. The potassium lactate is treated in the form of an aqueous solution. The concentration of potassium lactate in the solution to be treated is 40 to 80% by weight based on the combined weight of water and potassium lactate and the process can be carried out continuously or batch wise.

WO 03/031385 describes a method for making a powder of Na (or K) lactate. This reference does not describe using a concentration method for removing odor and flavor from potassium lactate.

In US 2,143,361 12-15% sodium lactate is concentrated to 65% and thereafter diluted to 50%. Although it has been described that potassium lactate can be concentrated in a similar manner, this reference does not disclose concentrating the aqueous solution to above 65%, not does it give any indication that such method could lead to improvement of odor and flavor.

In Systermans, Asia Pacific Food Industry, (6), 2000, p. 1-2 the specifications of the commercially available Purasal P and HiPurasal 60 have been described. This reference is silent on the methods to improve the quality of the HiPurasal 60 product.

During the production of lactic acid by fermentation, normally neutralizing agents are used to neutralize the fermentation broth. In, for instance, GB 907,321 it has been described that calcium sulfate is used as neutralizing agent so that calcium lactate is formed. Afterwards, the calcium ions are replaced by hydrogen using ion exchange but these can also be replaced by sodium or potassium ions. Solutions thus obtained are not suitable for food applications since they contain substantial amounts of impurities such as ethanol, acetaldehyde, polysaccharides, diacetyl, 2-butanone, 2,3-pentanedione, and 2-butenal which cause bad odor and bad flavor. The free lactic acid, not its potassium salt, may be subjected to further purification and concentration.

A potassium lactate product that is purified according to prior art methods is, for instance, PURASAL HiPure® P, which is commercially available as a 60% solution by Purac Biochem BV, the Netherlands. When converting lactic acid to potassium lactate, theoretically higher concentrations than 60% can be obtained in this manner, but practically not. The reaction is highly exothermic and any attempt to make higher concentrations would inevitably lead to unacceptable unsafe reaction conditions, and further to formation of side products due to the excessive high temperatures.

The purification of potassium lactate solutions with the active carbon method according to the prior art has several drawbacks. Firstly, the active carbon treatment is expensive due to its elaborated and time-consuming regeneration, which adds considerable to the costs of the product that needs to be cheap for its intended use. Secondly, as the quality of the regenerated carbon of this process fluctuates, the quality of the potassium lactate also fluctuates. Thirdly, the flavor and odor properties of this product are still susceptible to improvement.

It is therefore an objective of the present invention to provide a simpler method that provides aqueous potassium lactate solutions with improved organoleptic properties, and wherein carbon, if used, can more easily be regenerated. The resulting aqueous potassium lactate solution is in a form which is ready for further application in food and drink products and does not require any further complex processing technique.

To this end the invention pertains to a method for the preparation of an aqueous potassium lactate solution according to claim 1.

The invention also pertains to a method for the preparation of a food or drink product according to claim 2. The isolated product is directly applied in food and drink products or is packaged and shipped to customers for further applications in food and drink products. The product quality of the isolated product obtained by the present method was improved, as key aroma compounds appear to be removed from the product. The isolated product has a form which is suitable for further applications in various food and drink products, and particularly in meat and poultry, including fish.

Preferably, the potassium lactate solution is concentrated to a solution comprising 67 to 82 wt.%, more preferably 70 to 80 wt.%, and most preferably 76 to 79 wt.% of potassium lactate, since in above-mentioned concentration ranges a potassium lactate solution is obtained with an organoleptic profile that is optimal in terms of odor and taste sensation. Further, said product in above-mentioned ranges contains a minimum total concentration of key aroma compounds as ethanol, acetaldehyde, diacetyl, 2-butanone, 2,3-pentadione, and 2(E)-butenal. The total concentration of these key aroma compounds together is below 15 ppm, and even below 10 ppm for the 70 to 80 wt.% solutions. The ethanol concentration is less than 10 ppm for the above-mentioned potassium lactate concentration ranges and even below 5 ppm for solutions comprising 70 to 80 wt.% potassium lactate, which is a reduction of almost 10 times in comparison with commercially available potassium lactate solutions comprising 60 wt.% potassium lactate that were only carbon-treated.

Solutions with 76-79 wt.% potassium lactate content further show an optimum balance between the concentration and the viscosity of the potassium lactate solution. These solutions are therefore particularly efficient and suitable for further handling or application in food and drink products.

Since commercially available potassium lactate solutions commonly have a potassium lactate concentration of about 60 wt.%, it is preferred to use such solutions as the first (starting) potassium lactate solution.

In order to obtain higher quality products still having 60% potassium lactate, the first potassium lactate solution can be concentrated to a certain concentration between 65 and 85% and then diluted again to 60%.

Evaporation can be performed using any method for evaporation, such as distillation, flash distillation, wiped film distillation, short path distillation, vacuum distillation, rotary evaporation, spinning cone column evaporation, and the like. Sometimes it is preferred to perform the distillation in multiple, for instance two, separate distillation steps. In a first distillation step the first potassium lactate solution can be concentrated, for example, from 60% to 67%, and in a second distillation step this partially concentrated potassium lactate solution can be further concentrated from said 67% to, for example, 78%. If two distillation steps are used, it is further preferred to perform these steps in two different distillation units, but the evaporation can also take place in one apparatus because most evaporation apparatuses can be used as multi-step evaporator. As a suitable example the use of a plate evaporator is mentioned, wherein the liquid is pumped between thin plates with the heating medium on the mating surfaces, after which the product is evaporated with the vapor generated forming a high velocity core.

As explained, if possible, it is of advantage replacing the active carbon step by an evaporation step. However, due to coloring of the end product such step in some cases where further discolorization is desired may be still favorable. Such step can be performed prior to the evaporation step, for instance by using PURASAL HiPure P as the starting material, or alternatively the active carbon treatment can be performed after the evaporation step. It was found that such combined active carbon/evaporation method provides the highest quality end product. When performed prior to evaporation, it was found that a substantial improvement regarding taste and odor was achieved. When performed after evaporation, it was found that the regenerated carbon, and thus the resulting potassium lactate, showed less quality fluctuations.

It is a further objective of the invention to provide a method wherein a first potassium lactate solution having a potassium lactate concentration less than 65% is concentrated by evaporating said solution to a potassium lactate solution having a concentration from 65 to 85%, and diluting said solution again, for instance to below 65%, or to about 60%.

It is also an objective of the invention to provide an aqueous potassium lactate solution having a potassium lactate concentration from 65 to 85%, and which is free from or at least poor in bad flavor and/or odor. Such product is obtainable by the above evaporation method with or without the additional active carbon purification step.

Concentrations higher than 85 wt.% lead to viscous solutions that are less suitable and more difficult to handle in for example meat production where low temperatures such as 4 °C are used, but are still suitable for transport. The concentrated solutions as obtained according to the invention may be diluted to any diluted concentration, for instance to a solution comprising a potassium lactate concentration of less than 65 wt.% or of about 60 wt.%. The higher concentration solutions, however, have the additional advantage that bulk amounts can more easily be transported because these need less container space for the same amount of potassium lactate.

Preferred potassium lactate solutions have a potassium lactate concentration of 67 to 82 wt.%, more preferably of 70 to 80 wt.%. A particularly useful concentration is 76 to 79 wt.% having the concentration of potassium lactate as high as possible, resulting in a very efficient solution without coming in conflict with a too high viscosity of the solution. Above-mentioned solutions are very suitable for handling and application in food or drink products and particularly in meat products. Said solutions further have a very low profile with respect to the presence of the following key aroma compounds: acetaldehyde, ethanol, diacetyl, 2-butanone, 2,3-pentadione and 1(E)-butenal. All these compounds together are present in a total content of less than 10 ppm in potassium lactate solutions of 76-79 wt.% as obtainable according to the method of the present invention.

It was further found that solutions with potassium lactate concentrations between 65 and 85 wt.% as obtainable by the evaporation method according to the present invention contained an ethanol concentration of less than 10 ppm. Potassium lactate solutions of between 70 and 80 wt.% contained even less than 5 ppm of ethanol.

The invention is further illustrated by the following non-limitative examples.

### Experimental

Five samples containing 60% of potassium lactate were evaporated by SPD (short path distillation) to obtain a concentrated solution.

The temperature of the heating oil was varied in order to obtain different concentrations. Table 1 gives the final concentration of the samples.

**Table 1. Concentration of the samples**

| Sample | Tₒᵢₗ [°C] | Concentration [wt.%] |
|---|---|---|
| 1 | 100 | 67.1 |
| 2 | 115 | 70.6 |
| 3 | 145 | 78.1 |
| 4 | 160 | 82.1 |
| 5 | Reference* | 60.0 |

| | | |
|---|---|---|
| *PURASAL HiPure® P; ex Purac America Inc., USA | | |

### GC-MS results

The relative areas of key aroma compounds found by GC-MS are given in Table 2. The concentrated potassium lactate solutions were diluted to 60%, prior to analysis.

**Table 2. Relative areas of key aroma compounds**

| | | | | | |
|---|---|---|---|---|---|
| Concentration (%) | 60* | 67.1 | 70.6 | 78.1 | 82.1 |
| | | | | | |
| Acetaldehyde | 43.38 | 2.20 | 1.54 | 1.00 | 1.45 |
| Ethanol | 53.64 | 5.74 | 1.92 | 1.00 | 6.93 |
| Diacetyl | 3.46 | 1.00 | 1.03 | 1.34 | 1.92 |
| 2-Butanone | 37.21 | 1.00 | 1.18 | 1.06 | 1.14 |
| 2,3-Pentanedione | 7.79 | 1.22 | 1.09 | 1.00 | 1.51 |
| 2(E)-Butenal | 8.12 | 1.63 | 1.41 | 1.00 | 1.44 |
| Total | 153.60 | 12.78 | 8.17 | 6.40 | 14.39 |

| | | | | | |
|---|---|---|---|---|---|
| * reference = PURASAL HiPure P | | | | | |

### Taste and odor

Odor and taste were tested by a sensory panel. Four samples were selected based on the GC-MS results. These samples were compared with a sample P-Lac®, 60% potassium lactate (ex Wilke Resources Inc., USA).

The samples for the odor test were diluted to 60% dry solids. The samples for taste were tested by diluting the samples to 1.12% dry solids. 6 Panel members judged the samples on taste and 4 panel members judged the samples on odor. The samples were ranked from 1 (low) to 5 (high).

Tables 3 and 4 show the results of the odor and taste tests, respectively.

**Table 3. Results of odor**

| Sample | Concentration (wt.%) | Average ranking* |
|---|---|---|
| 1 | 67.1 | 2.0 |
| 3 | 78.1 | 2.5 |
| 5# | 60.0 | 3.0 |
| P-Lac® | 60.0 | 4.0 |

| | | |
|---|---|---|
| * 1:low intensity, 5:high intensity # PURASAL HiPure.P (reference) | | |

**Table 4. Results of taste: intensity and bitterness**

| Sample | Concentration (wt.%) | Average ranking Intensity* | Average ranking Bitterness* |
|---|---|---|---|
| 1 | 67.1 | 1.8 | 2.0 |
| 3 | 78.1 | 2.0 | 2.2 |
| 5# | 60.0 | 4.5 | 4.2 |
| P-Lac® | 60.0 | 3.2 | 3.0 |

| | | | |
|---|---|---|---|
| * 1:low intensity; 5:high intensity 5 panel members ranked the samples on bitterness # PURASAL HiPure® P (reference) | | | |

## Claims

1. A method for the preparation of an aqueous potassium lactate solution wherein a first potassium lactate solution having a potassium lactate concentration less than 65 wt.% is concentrated by evaporation to a solution having a potassium lactate concentration from 65 wt.% to 85 wt.%, which is subsequently isolated, and packaged and shipped for application in food or drink products.

2. A method for the preparation of a food or drink product wherein a first potassium lactate solution having a potassium lactate concentration less than 65 wt.% is concentrated by evaporation to a solution having a potassium lactate concentration from 65 wt.% to 85 wt.%, which is subsequently isolated and directly applied in food or drink products.

3. The method according to claim 1 or 2 wherein the first potassium lactate solution has a potassium lactate concentration of 60 wt.%.

4. The method according to any one of claims claim 1 to 3 wherein the evaporation is performed in two steps, optionally in two distillation apparatuses.

5. The method according to any one of claims 1 to 4 wherein the evaporation is performed by a plate evaporation process.

6. The method according to any one of claim 1 to 5 wherein the evaporation is preceded or followed by treatment of the solution with active carbon.

7. The method according to any one of the previous claims wherein the concentrated potassium lactate solution is diluted.

8. The method according to any one of claims 1-6, wherein the isolated product is applied in meat and poultry, including fish.

9. Potassium lactate solution obtainable by a method wherein a first potassium lactate solution having a potassium lactate concentration less than 65 wt.% is concentrated by evaporation to a solution having a potassium lactate concentration from 65 wt.% to 85 wt.%, said potassium lactate solution having an ethanol concentration less than 10 ppm, wherein the solution is packaged for use in food and drink products.

10. Food or drink product comprising a potassium lactate solution obtainable by a method wherein a first potassium lactate solution having a potassium lactate concentration less than 65 wt.% is concentrated by evaporation to a solution having a potassium lactate concentration from 65 wt.% to 85 wt.%, said potassium lactate solution having an ethanol concentration less than 10 ppm.

11. Food product according to claim 10, which is a meat or poultry product, including fish.

12. Use in food and drink products of a potassium lactate solution obtained by a method wherein a first potassium lactate solution having a potassium lactate concentration less than 65 wt.% is concentrated by evaporation to a solution having a potassium lactate concentration from 65 wt.% to 85 wt.%.

13. Use according to claim 12 wherein the food product is a meat or poultry product, including fish.

## Patentansprüche

1. Ein Verfahren zur Herstellung einer wässrigen Kaliumlactatlösung, wobei eine erste Kaliumiactatlösung mit einer Kaliumlactatkonzentration von weniger als 65 Gew.-% durch Verdampfung zu einer Lösung mit einer Kaliumlactatkonzentration von 65 Ges.-% bis 85 Gew.-% konzentriert wird, welche anschließend isoliert und verpackt und zur Anwendung in Lebensmittel- und Getränkeprodukten verschickt wird.

2. Ein Verfahren zur Herstellung eines Lebensmittel- oder Getränkeprodukts, wobei eine erste Kaliumlactatlösung mit einer Kaliumlactatkonzentration von weniger als 65 Gew.-% durch Verdampfung zu einer Lösung mit einer Kaliumlactatkonzentration von 65 Gew.-% bis 85 Gew.-% konzentriert wird, welche anschließend isoliert und in Lebensmittel- oder Getränkeprodukten direkt angewandt wird.

3. Das Verfahren gemäß Anspruch 1 oder 2, wobei die erste Kaliumlactatlösung eine Kaliumlactatkonzentration von 60 Gew.-% aufweist.

4. Das Verfahren gemäß einem der Ansprüche 1 bis 3, wobei die Verdampfung in zwei Schritten durchgeführt wird, gegebenenfalls in zwei Destillationsapparaturen.

5. Das Verfahren gemäß einem der Ansprüche 1 bis 4, wobei die Verdampfung durch ein Plattenverdampfungsverfahren durchgeführt wird.

6. Das Verfahren gemäß einem der Ansprüche 1 bis 5, wobei der Verdampfung eine Behandlung der Lösung mit Aktivkohle vorausgeht oder folgt.

7. Das Verfahren gemäß einem der vorangegangenen Ansprüche, wobei die konzentrierte Kaliumlactatlösung verdünnt wird.

8. Das Verfahren gemäß einem der Ansprüche 1 bis 6, wobei das isolierte Produkt bei Fleisch und Geflügel, einschließlich Fisch, angewandt wird.

9. Kaliumlactatlösung, erhältlich durch ein Verfahren, wobei eine erste Kaliumlactatlösung mit einer Kaliumlactatkonzentration von weniger als 65 Gew.-% durch Verdampfung zu einer Lösung mit einer Kaliumlactatkonzentration von 65 Gew.-% bis 85 Gew.-% konzentriert wird, wobei die Kaliumlactatlösung eine Ethanolkonzentration von weniger als 10 ppm aufweist, wobei die Lösung zur Verwendung in Lebensmittel- und Getränkeprodukten verpackt ist.

10. Lebensmittel- oder Getränkeprodukt, umfassend eine Kaliumlactatlösung, erhältlich durch ein Verfahren, wobei eine erste Kaliumlactatlösung mit einer Kaliumlactatkonzentration von weniger als 65 Gew.-% durch Verdampfung zu einer Lösung mit einer Kaliumlactatkonzentration von 65 Gew.-% bis 85 Gew.-% konzentriert wird, wobei die Kaliumlactatlösung eine Ethanolkonzentration von weniger als 10 ppm aufweist.

11. Lebensmittelprodukt gemäß Anspruch 10, welches ein Fleisch- oder Geflügelprodukt, einschließlich Fisch, ist.

12. Verwendung einer Kaliumlactatlösung, erhalten durch ein Verfahren, wobei eine erste Kaliumlactatlösung mit einer Kaliumlactatkonzentration von weniger als 65 Gew.-% durch Verdampfung zu einer Lösung mit einer Kaliumlactatkonzentration von 65 Gew.-% bis 85 Gew.-% konzentriert wird, in Lebensmittel- oder Getränkeprodukten.

13. Verwendung gemäß Anspruch 12, wobei das Lebensmittelprodukt ein Fleisch- oder Geflügelprodukt, einschließlich Fisch, ist.

## Revendications

1. Procédé de préparation d'une solution aqueuse de lactate de potassium dans lequel une première solution de lactate de potassium ayant une concentration en lactate de potassium inférieure à 65 % en poids est concentrée par évaporation pour obtenir une solution ayant une concentration en lactate de potassium de 65 à 85 % en poids, qui est ultérieurement isolée, puis conditionnée et expédiée à des fins d'application à des produits alimentaires ou de type boisson.

2. Procédé de préparation d'un produit alimentaire ou de type boisson dans lequel une première solution de lactate de potassium ayant une concentration en lactate de potassium inférieure à 65 % en poids est concentrée par évaporation pour obtenir une solution ayant une concentration en lactate de potassium de 65 à 85 % en poids, qui est ultérieurement isolée et directement appliquée à des produits alimentaires ou de type boisson.

3. Procédé selon la revendication 1 ou 2 dans lequel la première solution de lactate de potassium a une concentration en lactate de potassium de 60 % en poids.

4. Procédé selon l'une quelconque des revendications 1 à 3 dans lequel l'évaporation est réalisée en deux étapes, éventuellement dans deux appareils de distillation.

5. Procédé selon l'une quelconque des revendications 1 à 4 dans lequel l'évaporation est réalisée par un procédé d'évaporation du type à plaques.

6. Procédé selon l'une quelconque des revendications 1 à 5 dans lequel l'évaporation est précédée ou suivie du traitement de la solution avec du charbon actif.

7. Procédé selon l'une quelconque des revendications précédentes dans lequel la solution de lactate de potassium concentrée est diluée.

8. Procédé selon l'une quelconque des revendications 1 à 6, dans lequel le produit isolé est appliqué à la viande et à la volaille, y compris au poisson.

9. Solution de lactate de potassium pouvant être obtenue par un procédé dans lequel une première solution de lactate de potassium ayant une concentration en lactate de potassium inférieure à 65 % en poids est concentrée par évaporation pour obtenir une solution ayant une concentration en lactate de potassium de 65 à 85 % en poids, ladite solution de lactate de potassium ayant une concentration en éthanol inférieure à 10 ppm, et dans lequel la solution est conditionnée pour pouvoir être utilisée dans des produits alimentaires et de type boisson.

10. Produit alimentaire ou de type boisson comprenant une solution de lactate de potassium pouvant être obtenue par un procédé dans lequel une première solution de lactate de potassium ayant une concentration en lactate de potassium inférieure à 65 % en poids est concentrée par évaporation pour obtenir une solution ayant une concentration en lactate de potassium de 65 à 85 % en poids, ladite solution de lactate de potassium ayant une concentration en éthanol inférieure à 10 ppm.

11. Produit alimentaire selon la revendication 10, qui est un produit de type viande ou volaille, y compris le poisson.

12. Utilisation dans des produits alimentaires et de type boisson d'une solution de lactate de potassium obtenue par un procédé dans lequel une première solution de lactate de potassium ayant une concentration en lactate de potassium inférieure à 65 % en poids est concentrée par évaporation pour obtenir une solution ayant une concentration en lactate de potassium de 65 à 85 % en poids.

13. Utilisation selon la revendication 12 dans laquelle le produit alimentaire est un produit de type viande ou volaille, y compris le poisson.
